# NOUVEAU FASCICULE DE BREVET EUROPEEN

(11) **EP 0 267 059 B2**
(45) Date de publication et mention de la décision concernant l'opposition: **18.10.1995**
(45) Mention de la délivrance du brevet: 18.12.1991
(21) Numéro de dépôt: 87402017.5
(22) Date de dépôt: 10.09.1987
(51) Int. Cl.: A61F 13/15

(54) **Serviette périodique comportant des ailes ou volets latéraux qui sont soudés ensemble**
Monatsbinde, versehen mit aneinander geschweissten Flügeln oder Flächern
Sanitary napkin having tabs or flaps welded together

(30) Priorité: 06.10.1986 FR 8613867
(43) Date de publication de la demande: 11.05.1988
(73) Titulaire: KAYSERSBERG SA, F-68240 Kaysersberg (FR)
(72) Inventeur: Pigneul, Raymond, F-68320 Durrenentzen (FR); Ruppel, Rémy, F-68000 Horbourg (FR)
(74) Mandataire: David, Daniel

(56) Documents cités:
- EP-A- 0 134 086
- DE-A- 3 538 263
- FR-A- 708 828
- FR-A- 2 275 162
- FR-A- 2 350 830
- GB-A- 2 168 253
- US-A- 4 324 246

## Description

La présente invention concerne essentiellement un dispositif formant serviette périodique ou similaire comportant des ailes ou volets latéraux soudés ensemble améliorant l'étanchéité latérale, de préférence pourvus de plis.

On connaît déjà de multiples dispositifs formant serviette périodique ou similaire, en particulier pour l'hygiène féminine.

Par exemple, le brevet US 3 805 790 décrit un dispositif formant serviette périodique comprenant un élément tampon absorbant les fluides (16) préformé généralement biconcave, définissant une zone biconcave intermédiaire de largeur réduite.

Cet élément tampon (16) est disposé entièrement à l'intérieur d'une enveloppe définie par une pellicule supérieure (20) perméable aux fluides et un pellicule inférieure (18) imperméable aux fluides.

L'enveloppe est de dimensions adaptées à la forme de l'élément tampon absorbant de manière à l'enserrer étroitement sans jeu, et présente donc la même biconcavité.

On souligne que cette forme biconcave améliore le confort du patient (voir colonne 5, lignes 42 à 49).

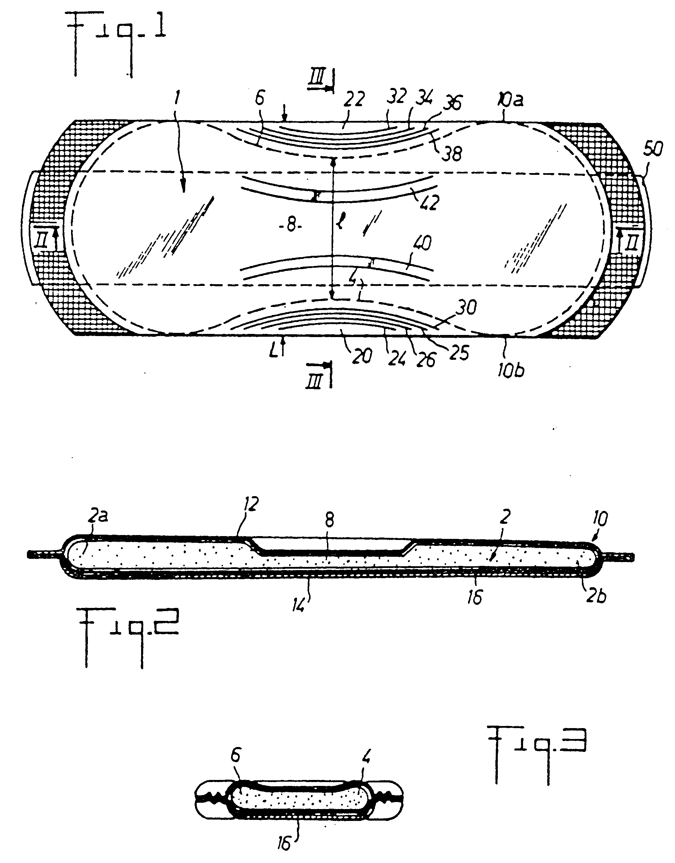

Le brevet US 2 331 355 révèle également un dispositif formant serviette périodique similaire, comportant en outre un ou plusieurs plis centraux permettant de définir une zone incurvée de réception de fluides. Les plis sont disposés dans la zone centrale longitudinale du dispositif (voir figures 1 à 28).

Le brevet US 3 203 419 décrit encore un dispositif formant serviette périodique ainsi qu'un procédé de fabrication de celui-ci.

Selon ce dispositif, l'élément tampon absorbant (10) est entièrement disposé à l'intérieur d'une enveloppe perméable aux fluides fermée à la réunion de deux pellicules respectivement inférieure et supérieure référencées (11), qui sont ensuite réunies ensemble par soudure (voir figure 3). La couche (16) de l'élément imperméable aux fluides est interposée entre un élément tampon (10) et l'enveloppe (11).

Selon ce dispositif, l'élément tampon présente une forme en coupe axiale longitudinale biconvexe et des bords latéraux droits (voir figure 2), ce qui est défavorable pour le confort et n'assure pas une bonne étanchéité latérale.

La demande européenne EP-A-67 377 décrit encore un dispositif formant serviette périodique comportant un élément tampon biconcave, dont la concavité est réalisée par une pré-compression de l'élément tampon, en vue d'améliorer le confort.

Le niveau de compression est tel que l'on diminue d'au moins 10 % la largeur de l'élément tampon au niveau de la compression.

Ici encore l'enveloppe (2) est strictement de la même dimension que l'élément tampon comprimé (voir figure 2).

Le brevet US-A-3 575 174 révèle encore un dispositif similaire selon lequel la serviette périodique est incurvée depuis la partie avant vers la partie arrière de manière à lui donner une forme de cuvette. Vers la partie avant et vers la partie arrière, on prévoit deux rainures transversales (22) ainsi que des rainures longitudinales (20) (voir figures 4 et 5).

La demande européenne EP-A-136 524 révèle un dispositif formant serviette périodique pourvue de rainures assurant une courbure du dispositif de manière produire et à améliorer la conformation du corps de la personne portant le dispositif.

Le brevet européen EP-B-137 725 révèle un dispositif formant serviette périodique comportant un élément tampon absorbant, plan allongé entouré par un bord périphérique de plus forte densité afin d'assurer une meilleure étanchéité.

La demande française FR-A-2275162 concerne une garniture pour couche dont le tampon absorbant est en forme de sablier et l'enveloppe à bords rectilignes.

La demande européenne EP-A-134086 décrit une serviette périodique à volets latéraux associés au tampon par au moins deux lignes de pliage permettant la formation d'une cuvette après repliement de l'extrémité des volets sur la face externe du sous-vêtement, et mise en place sur le corps.

La demande française FR-A-2350830 décrit des protections pour hygiène féminine dont le tampon absorbant est comprimé le long des lignes sensiblement axiales.

Tous les dispositifs connus, notamment ceux précédemment exposés, ne donnent pas encore complètement satisfaction en ce qui concerne l'étanchéité latérale bien que certains aient fourni des solutions satisfaisantes relativement au confort.

La présente invention a pour but principal de fournir un dispositif formant serviette périodique ou similaire assurant une étanchéité latérale accrue en vue d'empêcher toute fuite latérale de fluides.

Ainsi la présente invention fournit un dispositif formant serviette périodique ou similaire, en particulier pour l'hygiène féminine, comprenant un élément tampon absorbant les fluides, présentant une face supérieure, une face inférieure et des bords latéraux longitudinaux étant de forme biconcave de façon à définir une zone centrale de largeur réduite, disposé entièrement à l'intérieur d'une enveloppe, perméable auxdits fluides, et étant prévue d'une largeur supérieure à celle dudit élément tampon au niveau de ladite zone centrale de largeur réduite, les bords latéraux de ladite enveloppe étant rectilignes, une couche d'un élément imperméable aux fluides étant interposée entre la face inférieure de l'élément tampon et ladite enveloppe, caractérisé en ce que les parties supérieure et inférieure de l'enveloppe, situées au voisinage de ladite zone centrale, sont soudées entre elles, entre lesdits bords latéraux rectilignes et en dehors de l'élément tampon, de façon à former deux volets latéraux améliorant l'étanchéité latérale relativement auxdits fluides, avec plusieurs plis chacun, conformés en arcs concentriques à la concavité adjacente.

Selon une caractéristique préférée de l'invention, les plis sont soudés ensemble améliorant encore l'étanchéité latérale.

Selon un mode de réalisation particulier, la soudure des ailes ou volets latéraux, ainsi que des plis précités, est réalisée à l'aide d'une colle adhésive, de préférence du type "hot melt", c'est-à-dire devenant fluide à la chaleur.

Selon une autre caractéristique avantageuse, le dispositif selon l'invention comprend une ou plusieurs gorges réalisées dans la masse de l'élément tampon, dans la zone centrale définie entre les concavités latérales précitées.

Avantageusement, ces gorges sont aussi réalisées en arcs de cercle, de préférence concentriques aux concavités précitées.

Selon une autre caractéristique particulière, les plis précités ainsi que les gorges sont disposés symétriquement par rapport au plan de symétrie longitudinal de la serviette périodique.

Les gorges précitées peuvent être réalisées par tout moyen approprié. De préférence les gorges sont cependant réalisées par compression du tampon absorbant, par tout moyen de compression approprié, tel que galet presseur.

D'autres buts, caractéristiques et avantages apparaîtront clairement à la lumière de la description explicative qui va suivre et fait référence aux dessins annexés, représentant un mode de réalisation actuellement préféré de l'invention, donné simplement à titre d'illustration, et qui ne saurait donc en aucune façon limiter la portée de la présente invention.

Dans les dessins :
- la figure 1 est une vue de dessus du dispositif formant serviette périodique actuellement préféré selon l'invention ;
- la figure 2 est une vue en coupe longitudinale selon la ligne de trace II-II, passant par une gorge de la zone centrale ;
- la figure 3 est une vue en coupe transversale selon la ligue de trace III-III de la figure 1.

En référence aux figures 1 à 3, un dispositif formant serviette périodique ou similaire selon l'invention, représenté par le numéro de référence général 1 comprend un élément tampon (2), absorbant les fluides, préformé, généralement biconcave par la présence de bords latéraux (4, 6) concaves, définissant une zone centrale (8) biconcave de largeur réduite par rapport aux zones d'extrémités respectives avant (2a) et arrière (2b).

Cet élément tampon (2) est disposé selon l'exemple de réalisation représenté entièrement à l'intérieur d'une enveloppe (10) perméable auxdits fluides, qui peut être définie par exemple par une liaison d'une pellicule supérieure (12) perméable aux fluides et d'une pellicule inférieure (14) pouvant être également perméable aux fluides.

Lorsque la pellicule inférieure (14) est perméable aux fluides, celle-ci est réalisée identique à la pellicule (12) et peut provenir d'une bande unique qui est conformée en enveloppe pour la réception à l'intérieur de l'élément tampon (2).

On obtient ainsi une simplification du procédé de fabrication.

De tels procédés sont bien connus et décrits par exemple dans le brevet US 3 203 419 et le brevet US 3 575 174, précédemment cités.

Ce dispositif comprend aussi généralement une couche (16) d'un élément imperméable aux fluides interposé entre l'élément tampon (2) et l'enveloppe (10) habituellement entre l'élément tampon (2) et la pellicule inférieure (14), qui est opposée à la pellicule supérieure (12) destinée à recevoir les fluides précités.

Selon la présente invention, l'enveloppe (10) est prévue d'une largeur (L) supérieure à celle (l) de l'élément tampon (2) au niveau de la zone biconcave (8), en définissant ainsi des ailes ou volets latéraux (20, 22) qui sont soudés ensemble. En dehors de la zone biconcave, l'enveloppe est adaptée à la forme de l'élément tampon (2) de manière à l'enserrer étroitement sans jeu.

Ainsi on améliore de manière radicale l'étanchéité latérale du dispositif relativement aux fluides, ce qui interdit toute fuite accidentelle de fluides.

On observera que de manière simple, ces ailes ou volets latéraux (20, 22) sont obtenus en prévoyant une enveloppe (10) avec des bords rectilignes (10a, 10b) comme cela est clairement visible à la figure 1.

De préférence, les ailes ou volets latéraux (20, 22) comprennent un ou plusieurs plis respectivement (24, 26, 28, 30) pour l'aile ou volet (20) et (32, 34, 36, 38) pour l'aile ou volet (22).

Ces plis (24 à 38) sont soudés ensemble, ce qui améliore encore l'étanchéité latérale. La soudure des ailes ou volets latéraux et des plis est de préférence obtenue à l'aide d'une colle adhésive, avantageusement du type "hot melt" de sorte qu'ils sont réalisés automatiquement lors de la soudure de la pellicule supérieure (12) avec la pellicule inférieure (14).

Les plis peuvent être aisément réalisés par la présence de galets formeurs gravés, c'est-à-dire comportant des rainures de forme appropriée, de tels types de galets étant bien connus par l'homme de l'art.

Selon une caractéristique particulièrement avantageuse de l'invention, les plis (20-38) sont conformés en arcs de cercle concentriques à la concavité adjacente.

Ainsi, les plis (24, 26, 28, 30) sont concentriques à la concavité adjacente (4) tandis que les plis (32, 34, 36, 38) sont concentriques à la concavité adjacente (6).

On comprend ainsi qu'en raison de la biconcavité du dispositif, ces plis soient réalisés symétriquement par rapport au plan de symétrie longitudinal du dispositif.

Selon une autre caractéristique du dispositif selon l'invention, celui-ci comprend une ou plusieurs gorges (40, 42) réalisées dans la masse de l'élément tampon (2), dans la zone centrale (8) définie entre les concavités latérales (4, 6).

Avantageusement, ces gorges (40, 42) sont elles-mêmes réalisées en arcs de cercle, de préférence concentriques aux concavités adjacentes respectivement (4) et (6), de manière à ce que l'élément soit disposé symétriquement par rapport au plan de symétrie longitudinal du dispositif.

Ces gorges (40, 42) sont avantageusement réalisées par compression du tampon absorbant (2) avec tout moyen de compression approprié, tel que le galet presseur comportant un épaulement de forme correspondante, comme cela est clairement compréhensible à l'homme de métier.

La présence de ces gorges (40, 42) est avantageuse en combinaison avec les ailes ou volets latéraux pour impartir une tendance à une déformation en cuvette du dispositif formant serviette périodique lors des mouvements de la personne portant le dispositif, en facilitant la réception des fluides précités et leur écoulement en direction de la partie centrale du dispositif.

On comprend ainsi qu'on obtient avec l'invention un dispositif formant serviette périodique garantissant pratiquement une étanchéité latérale absolue, tout en assurant un excellent confort.

Naturellement, l'invention comprend tous les moyens constituant des équivalents techniques des moyens décrits ainsi que leurs diverses combinaisons.

On comprend ainsi aisément que les dimensions respectives de l'élément constitutif du dispositif puissent être variables, de même la nature des matériaux constituant les divers éléments peut être classique ou être particulière en fonction d'un emploi particulier. En général, les éléments sont réalisés en des matériaux classiques, par exemple l'élément tampon (2) est réalisé en mousse de cellulose dans laquelle on peut incorporer des polymères hydrophiles insolubles ayant un excellent pouvoir d'absorption.

La couche (16) constituant l'élément imperméable aux fluides peut être réalisée en un matériau plastique, tel que le polyéthylène ou similaire.

Aussi, l'enveloppe peut être réalisée en un voile tissé ou non tissé perméable aux fluides, qui peut être hydrophobe, comme cela est bien connu.

De même, on peut prévoir extérieurement à la pellicule inférieure (14) une couche adhésive, avantageusement du type "hot melt", sur laquelle est collée une bande de protection (50) détachable lors de l'emploi, afin d'en faciliter la fixation à un sous-vêtement, comme cela est également bien connu.

Enfin, la couche (16) imperméable aux fluides peut être soudée ou collée à la pellicule inférieure (14) de l'enveloppe ou même faire partie intégrante de celle-ci et donc constituer cette pellicule inférieure (14).

## Revendications

1. Dispositif formant serviette périodique ou similaire, en particulier pour l'hygiène féminine, comprenant un élément tampon (2) absorbant les fluides, présentant une face supérieure, une face inférieure et des bords latéraux longitudinaux (4, 6) de forme bi-concave de façon à définir une zone centrale (8) de largeur réduite, ledit tampon étant disposé entièrement à l'intérieur d'une enveloppe (10), perméable auxdits fluides, ladite enveloppe étant prévue d'une largeur supérieure à celle dudit élément tampon au niveau de ladite zone centrale de largeur réduite, les bords latéraux (10a, 10b) de ladite enveloppe étant rectilignes, une couche (16) d'un élément imperméable aux fluides étant interposée entre ladite face inférieure de l'élément tampon (2) et ladite enveloppe (10), caractérisé en ce que les parties supérieure et intérieure de l'enveloppe, situées au voisinage de ladite zone centrale, sont soudées entre elles entre lesdits bords latéraux rectilignes (10a, 10b) et en dehors de l'élément tampon, de façon à former deux volets latéraux (20, 22) améliorant l'étanchéité latérale relativement auxdits fluides, avec plusieurs plis (24 à 38) chacun, conformés en arcs concentriques à la concavité adjacente (4) ou (6).

2. Dispositif selon la revendication 1, caractérisé en ce que les plis précités (24 à 38) sont soudés ensemble.

3. Dispositif selon la revendication 1, caractérisé en ce que la soudure des ailes ou volets latéraux (20, 22) et des plis précités (24 à 38) est réalisée à l'aide d'une colle adhésive, de préférence de type "holt-melt".

4. Dispositif selon l'une des revendications précédentes, caractérisé en ce qu'il comprend une ou plusieurs gorges (40, 42) réalisées dans la masse de l'élément tampon (2), dans la zone centrale (8) définie entre les concavités précitées (4, 6).

5. Dispositif selon l'une quelconque des revendications précédentes, caractérisé en ce que les gorges (40, 42) sont réalisées en arcs de cercle, de préférence concentriques aux concavités précitées (4) ou (6).

6. Dispositif selon la revendication 4 ou 5, caractérisé en ce que les gorges (40, 42) sont réalisées par compression du tampon absorbant, par tout moyen de compression approprié, tel que galet presseur.

7. Dispositif selon l'une quelconque des revendications 1 à 6, dans lequel l'enveloppe (10) est définie par une pellicule supérieure (12) et une pellicule inférieure (14), caractérisé en ce que la couche (16) imperméable aux fluides est soudée ou collée à la pellicule inférieure (14), ou fait partie intégrante de celle-ci en constituant ainsi la pellicule inférieure (14).

## Claims

1. Means forming a sanitary towel or the like, in particular for feminine hygiene, comprising a fluid-absorbent pad member (2) which has an upper side, a lower side and longitudinal lateral edges (4, 6) of biconcave shape so as define a central zone (8) of reduced width, the said pad being entirely located with an envelope (10) which is permeable to the said fluids, the said envelope having a width greater than that of the said pad member in the vicinity of the said central zone of reduced width, the lateral edges (10a, 10b) of the said envelope being straight, a layer (16) of a member which is impermeable to fluids being placed between the said underside of the pad member (2) and the said envelope (10), characterised in that the upper and lower parts of the envelope in the vicinity of the said central zone are welded together between the said straight lateral edges (10a, 10b) and outside the pad member in such a way as to form two lateral flaps (20, 22) improving lateral sealing with respect to the said fluids, with a plurality of folds (24 to 38) each in the form of arcs concentric with the adjacent concavity (4) or (6).

2. Means according to claim 1, characterised in that the aforesaid folds (24 to 38) are welded together.

3. Means according to claim 1, characterised in that the lateral wings or flaps (20, 22) and the aforesaid folds (24 to 38) are welded using an adhesive preferably of the hot melt type.

4. Means according to any one of the foregoing claims, characterised in that it comprises one or more grooves (40, 42) constructed within the mass of the pad member (2) in the central zone (8) defined between the aforesaid concavities (4, 6).

5. Means according to any one of the foregoing claims, characterised in that the grooves (40, 42) are constructed as arcs of a circle, which are preferably concentric with the aforesaid cavities (4) or (6).

6. Means according to claim 4 or 5, characterised in that the grooves (40, 42) are constructed by compressing the absorbent pad by any appropriate means of compression such as a pressing roller.

7. Means according to any one of claims 1 to 6, in which the envelope (10) is defined by an upper film (12) and a lower film (14), characterised in that the layer (16) which is impermeable to fluids is welded or bonded to the lower film (14) or forms an integral part thereof thus constituting the lower film (14).

## Patentansprüche

1. Vorrichtung in Form einer Monatsbinde oder dergleichen, insbesondere für die weibliche Hygiene, mit einem die Flüssigkeiten absorbierenden Tamponelement (2), das eine Oberseite, eine Unterseite und längs verlaufende Seitenränder (4, 6) bikonkaver Form aufweist, derart, daß ein zentraler Bereich (8) verringerter Breite gebildet wird, wobei das Tampon vollständig innerhalb einer flüssigkeitsdurchlässigen Hülle (10) angeordnet ist, die Hülle eine größere Breite als das Tamponelement auf Höhe des zentralen Bereiches verringerter Breite hat, die Seitenränder (10a, 10b) der Hülle geradlinig sind, eine Schicht (16) aus einem flüssigkeitsundurchlässigen Element zwischen der Unterseite des Tamponelementes (2) und der Hülle (10) angeordnet ist, dadurch gekennzeichnet, daß der obere und untere Teil der Hülle, die in Nähe des zentralen Bereiches angeordnet sind, zwischen den geradlinigen seitlichen Rändern (10a, 10b) und außerhalb des Tamponelementes so miteinander verschweißt sind, daß zwei flügelartige Seitenteile (20, 22) entstehen, die die seitliche Abdichtung gegenüber den Flüssigkeiten weiter verbessern, jeweils versehen mit mehreren Falten (24 bis 38) auf zu der benachbarten Einbuchtung (4) oder (6) konzentrischen Bögen.

2. Vorrichtung nach Anspruch 1, dadurch gekennzeichnet, daß die Falten (24 bis 38) miteinander verschweißt sind.

3. Vorrichtung nach Anspruch 2, dadurch gekennzeichnet, daß die Verschweißung der Seitenteile (20, 22) und der Falten (24 bis 38) mit Hilfe eines Klebers, vorzugsweise eines Heißschmelzklebers, erfolgt.

4. Vorrichtung nach einem der vorhergehenden Ansprüche, dadurch gekennzeichnet, daß sie eine oder mehrere Rillen (40, 42) aufweist, die im Material des Tamponelementes (2) in dem zentralen Bereich (8) zwischen den Einbuchtungen (4, 6) gebildet sind.

5. Vorrichtung nach Anspruch 4, dadurch gekennzeichnet, daß die Rillen (40, 42) in Kreisbögen verlaufen, die vorzugsweise konzentrisch zu den Einbuchtungen (4 oder 6) sind.

6. Vorrichtung nach Anspruch 4 oder 5, dadurch gekennzeichnet, daß die Rillen (40, 42) durch Kompression des absorbierenden Tamponelementes gebildet sind, und zwar durch geeignete Kompressionsmittel wie eine Druckrolle.

7. Vorrichtung nach einem der Ansprüche 1 bis 6, bei der die Hülle (10) von einer oberen Folie (12) und einer unteren Folie (14) gebildet wird, dadurch gekennzeichnet, daß die flüssigkeitsundurchlässige Schicht (16) mit der unteren Folie (14) verschweißt oder verklebt ist oder ein integraler Bestandteil derselben ist, so daß sie auf diese Weise die untere Folie (14) bildet.
